# EUROPEAN PATENT APPLICATION

(11) **EP 4 840 969 A2**
(43) Date of publication of application: **18.11.2026**
(21) Application number: 26195766.6
(22) Date of filing: 16.06.2017
(51) Int. Cl.: C12Q 1/6869

(54) **METHODS AND MATERIALS FOR THE EFFECTIVE USE OF COMBINED TARGETED ENRICHMENT OF GENOMIC REGIONS AND LOW COVERAGE WHOLE GENOME SEQUENCING**

(30) Priority: 17.06.2016 US 201662351742 P
(62) Document number(s) of the earlier application(s) in accordance with Art. 76 EPC: 17814148.7 / 3 472 356
(71) Applicant: Mayo Foundation for Medical Education and Research, Rochester, MN 55905 (US)
(72) Inventor: KOCHER, Jean-Pierre A., Rochester, Minnesota 55906-4083 (US); WANG, Chen, Rochester, Minnesota 55906-3943 (US)
(74) Representative: Fish & Richardson P.C.

(57) **Abstract**

This document provides methods and materials for using low coverage whole genome sequencing techniques to assess genomes. For example, methods and materials for using targeted nucleic acid amplification and/or capture techniques in combination with low coverage whole genome sequencing techniques to obtain high coverage sequencing data for one or more pre-selected regions of a genome are provided.

## Description

### CROSS-REFERENCE TO RELATED APPLICATIONS

This application claims priority to U.S. Application Serial No. 62/351,742, filed on June 17, 2016. The disclosure of the prior application is considered part of the disclosure of this application, and is incorporated in its entirety into this application.

### BACKGROUND

### 1. Technical Field

This document relates to methods and materials involved in using low coverage whole genome sequencing (LC-WGS) techniques to assess genomes. For example, this document provides methods and materials for performing targeted enrichment of genomic regions (e.g., targeted amplification and/or targeted capture techniques) in combination with LC-WGS techniques to assess genomes.

### 2. Background Information

High coverage whole genome sequencing techniques, which could theoretically be used to call variants, amplifications, and deletions genome wide, is currently not used in clinical applications due to the high cost of the test as well as the complexity of interpreting results. One whole genome sequencing assay used for clinical application is the LC-WGS assay that has a coverage of about 1x or less. LC-WGS was used successfully for the non-invasive screening of fetuses to report trisomy of chromosome 13, 18, and 21.

### SUMMARY

This document provides methods and materials for using low coverage whole genome sequencing techniques to assess genomes. For example, this document provides methods and materials for using targeted nucleic acid amplification and/or targeted nucleic acid capture techniques in combination with low coverage whole genome sequencing techniques to obtain high coverage sequencing data for one or more pre-selected regions of a genome. Generally, during whole genome sequencing, DNA is fragmented into short fragments that are about 400 to 500 base pairs long. About 100 to 150 base pairs are sequenced at one or both ends of these fragments. A sequenced section of a DNA fragment is called a sequence read. Coverage refers to the number of reads spanning over a specific genomic location. A sample sequenced at 10x average coverage means that, on average, 10 reads span the genomic regions that were sequenced.

As described herein, combining targeted nucleic acid amplification and/or targeted nucleic acid capture techniques with low coverage whole genome sequencing techniques can generate a sequencing coverage that is less than about 1x for the regions of the genome outside the one or more pre-selected regions amplified and/or captured and a sequencing coverage that is greater than about 500x for the one or more pre-selected regions. For example, combining targeted nucleic acid amplification and/or targeted nucleic acid capture techniques with low coverage whole genome sequencing can provide a composite low resolution view of genomic variations across the genome with a high resolution view of genomic variations in one or more selected regions that were enriched via nucleic acid amplification and/or nucleic acid capture techniques. This can allow clinicians to obtain high coverage sequencing data for one or more pre-selected regions of a genome while performing cost effective, low coverage whole genome sequencing.

In general, one aspect of this document features a method for increasing the number of sequencing reads of one or more pre-selected genomic regions using low coverage whole genome sequencing. The method comprises, or consist essentially of, performing an amplification reaction using a genomic nucleic acid sample to amplify one or more pre-selected genomic regions, thereby forming an amplified sample, and performing low coverage whole genome sequencing using the amplified sample, wherein the coverage of the pre-selected genomic regions using the low coverage whole genome sequencing is greater than 250x, and wherein the coverage of regions outside the pre-selected genomic regions using the low coverage whole genome sequencing is less than 10x, less than 5x, or less than 3x. The one or more pre-selected genomic regions can be from one pre-selected genomic region to 2500 pre-selected genomic regions. The one or more pre-selected genomic regions can be from one pre-selected genomic region to 2000 pre-selected genomic regions. The one or more pre-selected genomic regions can be from one pre-selected genomic region to 1500 pre-selected genomic regions. The low coverage whole genome sequencing can be whole genome sequencing with less than 2x genome wide coverage. The low coverage whole genome sequencing can be whole genome sequencing with less than 1x genome wide coverage. The genomic nucleic acid sample can be a human genomic nucleic acid sample. The coverage of the pre-selected genomic regions using the low coverage whole genome sequencing can be greater than 500x. The coverage of the pre-selected genomic regions using the low coverage whole genome sequencing can be greater than 1000x (or greater than 1500x, greater than 2000x, greater than 3000x, greater than 5000x, greater than 7500x, or greater than 10000x). The method can comprise performing the amplification reaction using the genomic nucleic acid sample to amplify one or more pre-selected genomic regions having a length from about 150 bp to about 750 bp.

In another aspect, this document features a method for increasing the number of sequencing reads of one or more pre-selected genomic regions using low coverage whole genome sequencing. The method comprises, or consists essentially of, performing a nucleic acid capture reaction using a genomic nucleic acid sample to enrich one or more pre-selected genomic regions, thereby forming an enriched sample, and performing low coverage whole genome sequencing using the enriched sample, wherein the coverage of the pre-selected genomic regions using the low coverage whole genome sequencing is greater than 250x, and wherein the coverage of regions outside the pre-selected genomic regions using the low coverage whole genome sequencing is less than 10x, less than 5x, or less than 3x. The one or more pre-selected genomic regions can be from one pre-selected genomic region to 2500 pre-selected genomic regions. The one or more pre-selected genomic regions can be from one pre-selected genomic region to 2000 pre-selected genomic regions. The one or more pre-selected genomic regions can be from one pre-selected genomic region to 1500 pre-selected genomic regions. The low coverage whole genome sequencing can be whole genome sequencing with less than 2x genome wide coverage. The low coverage whole genome sequencing can be whole genome sequencing with less than 1x genome wide coverage. The genomic nucleic acid sample can be a human genomic nucleic acid sample. The coverage of the pre-selected genomic regions using the low coverage whole genome sequencing can be greater than 500x. The coverage of the pre-selected genomic regions using the low coverage whole genome sequencing can be greater than 1000x (or greater than 1500x, greater than 2000x, greater than 3000x, greater than 5000x, greater than 7500x, or greater than 10000x). The method can comprise performing the nucleic acid capture reaction using the genomic nucleic acid sample to capture one or more pre-selected genomic regions having a length from about 150 bp to about 750 bp.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DESCRIPTION OF THE DRAWINGS

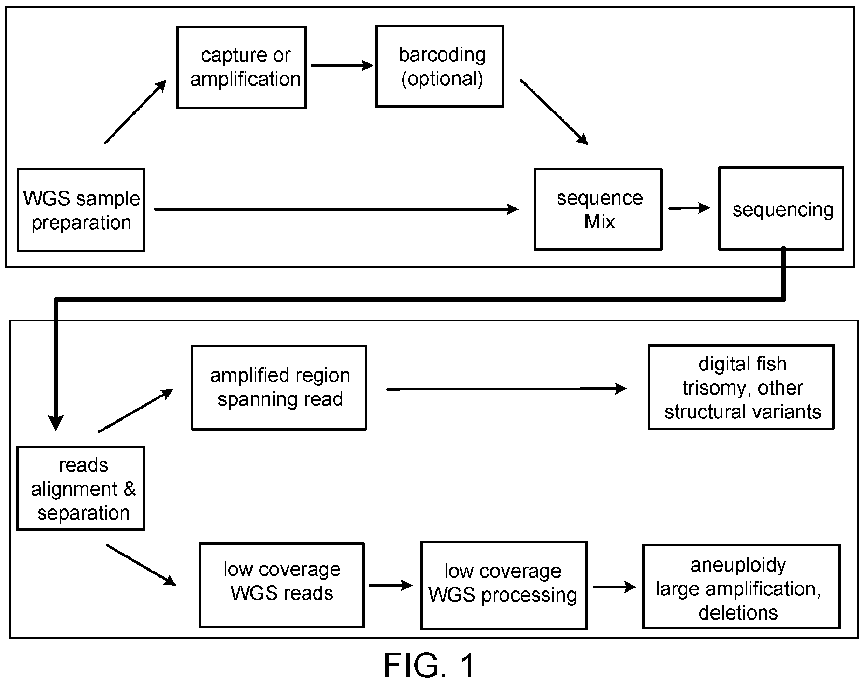

Figure 1 is a schematic of the steps of an exemplary workflow for the processing of a sequencing protocol according to one embodiment.

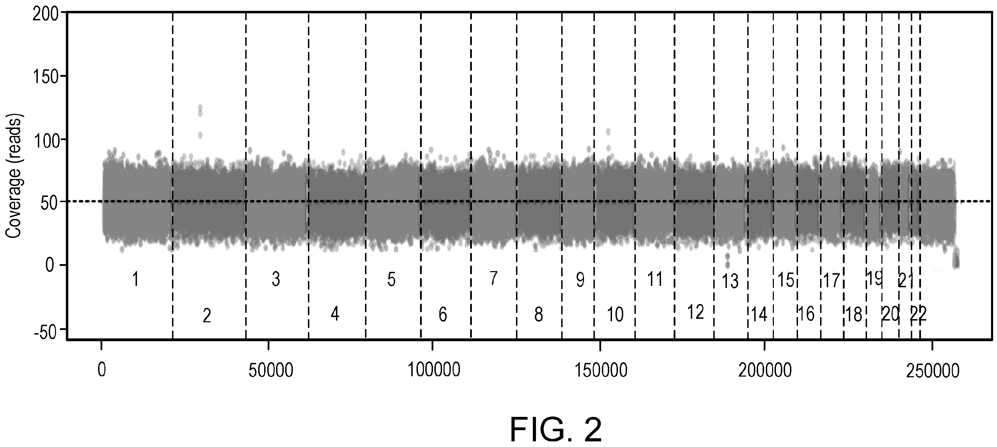

Figure 2 is a graph plotting LC-WGS sequencing coverage of a normal sample. The X axis displays the coverage on each chromosome that are numbered in ascending order. The Y axis is the number of reads mapped to the genomic region associated to a bin. Bioinformatics techniques are applied to the data to optimize evenness of coverage across the genome (X axis). Each dot on the plot represents a bin of 10kb. In this example, bins include in average 50 reads, but fluctuate between 10x and 80x. In this sample, no statistically significant amplifications or deletions are observed.

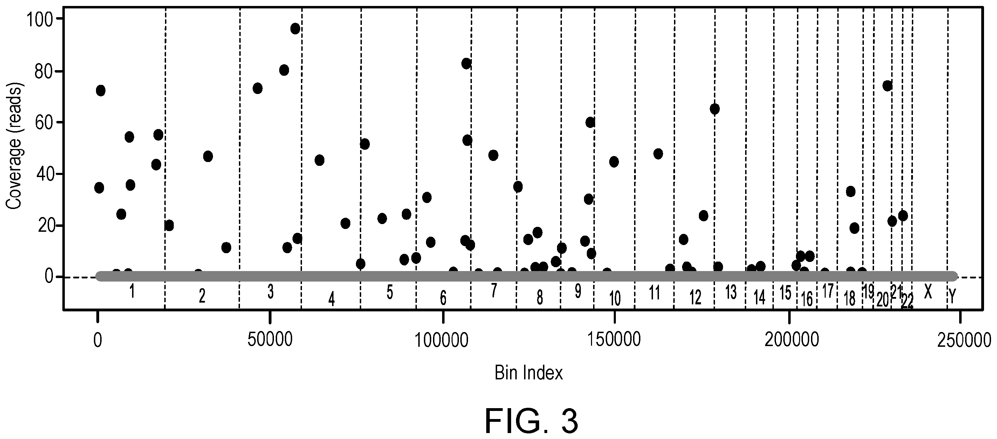

Figure 3 is a graph plotting the sequencing results obtained by combining the use of low coverage whole genome sequencing and amplification of selected regions. The X axis displays the coverage on each chromosome that are numbered in ascending order. The Y axis is the number of reads divided by 1000 that are mapped to the genomic region associated to a bin. The sample sequenced is a normal sample. Each circle represents a bin of 10kb. The LC-WGS is represented by set of grey circles that form a base line due to the scale of the plot. On average, 50 reads of 150bp are found in each bin. The black circles represent the coverage level of 97 loci that are 90 bases long and that were amplified using a PCR assay (amplicon). The coverage of these loci can reach for some of them 100,000x and therefore can be used to call genotypes, identify somatic mutations, identify breakpoints associated to structural variants or identify change of coverage informative of the amplification or deletion of these regions. In this example, the amplified regions overlap with SNPs from which the genotypes can be called accurately. The genotypes of SNPs cannot be called from low coverage sequencing alone.

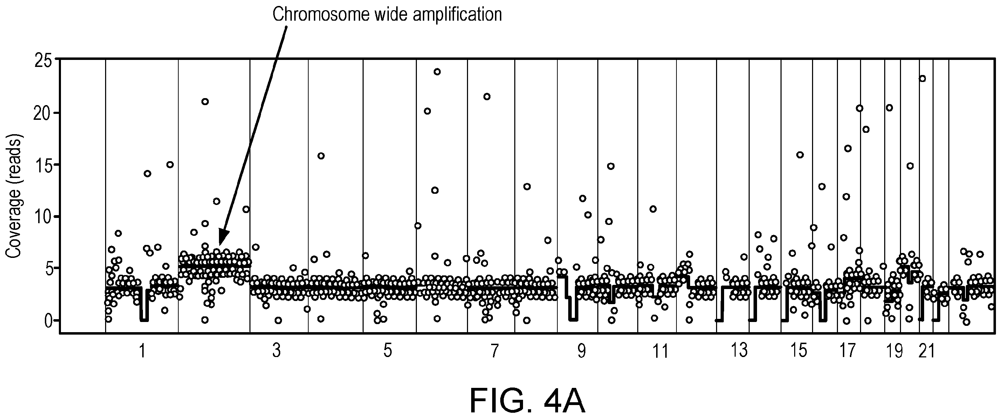

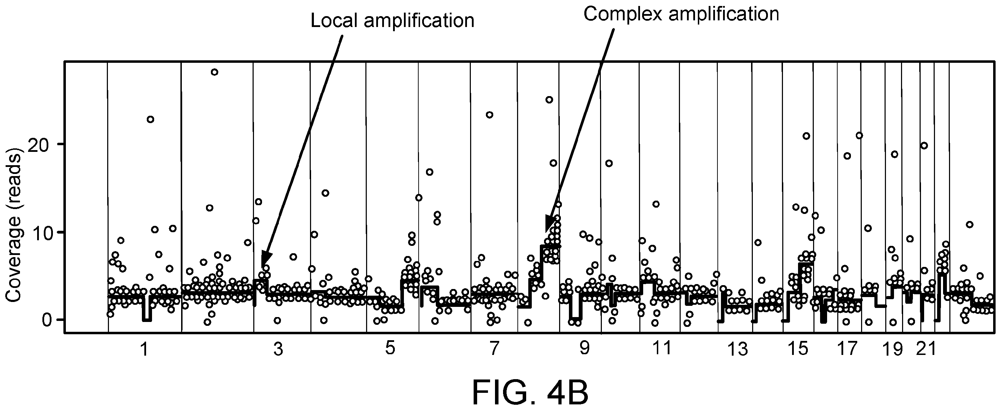

Figure 4A and 4B. LC-WGS coverage computed from the reads extracted from a targeted amplification assay where PCR amplification was performed below saturation. No-coverage regions correspond to centromers. Chromosome wide amplification, local and complex patterns of amplification are clearly visible in these plots.

### DETAILED DESCRIPTION

This document provides methods and materials for using low coverage whole genome sequencing techniques to assess genomes (e.g., genomic variations). For example, this document provides methods and materials for using targeted nucleic acid amplification and/or targeted nucleic acid capture techniques in combination with low coverage whole genome sequencing techniques to obtain high coverage sequencing data (e.g., over 500x coverage) for one or more selected regions of a genome.

Low coverage whole genome sequencing can be performed by limiting the concentration of DNA input in the sequencing reaction. A sample from a healthy human and assessed using low coverage whole genome sequencing without enriching pre-selected regions can be as shown in Figure 2. In some cases, samples can be multiplexed in a single whole genome sequencing assay. The concentration of each sample can be controlled to ensure that the DNA concentration is proportional to the number of samples. For example, the Illumina HiSeq 2000 can be set to produce per lane of flow cell: 300,000,000 reads that are 100 base pair long. Since the human genome is about 3 billion bases long, the whole genome of a single sample could be sequenced with a coverage of 10x coverage. If 10 samples are sequenced together in a flow cell lane, then the coverage per sample will be on average about 1x.

As described herein, combining targeted nucleic acid amplification and/or capture techniques with low coverage whole genome sequencing techniques can generate a sequencing coverage that is from less than about 1x coverage for the regions of the genome outside the one or more selected regions amplified and/or captured and a sequencing coverage that can be greater than 50,000x for the one or more selected regions (see, e.g., Figure 3).

Any appropriate nucleic acid amplification technique can be used to increase the sequence read coverage of one or more selected regions targeted for amplification. For example, PCR amplification can be used to increase the sequence read coverage of one or more selected regions when low coverage whole genome sequencing is used. In some cases, nucleic acid amplification techniques can be used to amplify more than 2000 regions of a genome. Increasing the number of amplified regions decreases the number of reads available to cover the whole genome and therefore decreases the LC-WGS coverage.

In some cases, nucleic acid capture techniques can be used in addition to, or in place of, nucleic acid amplification techniques to increase the sequence read coverage of one or more selected regions targeted for enrichment. Any appropriate nucleic acid capture technique can be used to increase the sequence read coverage of one or more selected regions targeted for enrichment. For example, DNA can be used as bait to capture a targeted sequence as described elsewhere (Hagemann et al., Cancer Genetics, 206:420-431 (2014)).

In some cases, in a single experimental protocol, a low coverage whole genome sequencing assay can be combined with a targeted amplicon assay, where PCR is used to amplify selected regions of the genome. In some cases, the amplification step can be replaced with a nucleic acid capture technique to capture genomic regions that can be combined with a low coverage whole genome sequencing assay. The sequencing result can be a combination of low coverage whole genome sequencing that provides an overview of the genomic amplification/deletion (e.g., duplications or other genomic amplifications or genomic deletions) landscape of the genome with high coverage sequencing data for the amplified and/or captured regions (e.g., a coverage up to several 1000x; see, e.g., Figure 3). This high coverage sequencing data obtained using an otherwise low coverage whole genome sequencing assay can be used to identify single nucleotide variants, indels, translocations, and/or copy number changes at a high sensitivity. For example, selected genomic regions can be selected and enriched (e.g., amplified) so that high coverage is obtained for those regions to identify SNPs, genomic amplifications, genomic deletions, and translocations. In some cases, the high sensitivity in these regions can be set to be similar to that obtained using fluorescence *in situ* hybridization (FISH) techniques.

Briefly, one exemplary implementation of the methods provided herein can include the following steps: (a) DNA extraction, (b) an optional whole genome amplification step if enough DNA is not available, (c) PCR amplification of one or more targeted genomic regions with a controlled number of PCR cycles, (d) optional genomic barcoding if multiple samples are to be sequenced in a single experiment, and (e) low coverage whole genome sequencing. Other exemplary implementations of the methods provided herein can be carried out as set forth in Figure 1.

Since the amount of DNA sequenced is about constant per sequencing experiment, the number and length of the genomic regions to be amplified, the coverage level expected for these regions, and the number of samples to be sequenced in a single experiment can be directly related to the sequencing reads left to cover the whole genome.

The following parameters can be used to design an assay provided herein such that it achieves a particular coverage for the genomic regions enriched and those genomic regions not enriched: (a) total number of reads produced by the sequencing platform, (b) number of samples to sequence in a single experiment, (c) number of target regions to amplify or capture, (d) length of the region to amplified or captured, and (e) expected coverage of the enriched target regions.

The following defines the relationship between these parameters: $LC = \left(RS*RL-AN*AL*AC\right) / LG$ where:
RS is the number of sequenced read per sample
RL is the length of a read (in bases)
AN is the number of amplicons
AL is the length of the amplicons (in bases)
AC is the coverage of each amplicons
LC is the coverge of the LC-WGS
LG is the number of base pair in the sequenced genome

Table 1 sets forth different exemplary combinations of the parameters RL, AN, AL, AC, and LC. LG is set to 3 billion base pairs (human genome)

**Table 1.**

| reads per sample | read length | number of amplicons | amplicon length | amplicon coverage | LC-WGS coverage |
|---|---|---|---|---|---|
| (RS) | (RL) | (AN) | (AL) | (AC) | (LC) |
| 30,000,000 | 100 | 100 | 500 | 5,000 | 0.92 |
| 30,000,000 | 100 | 100 | 1000 | 5,000 | 0.83 |
| 30,000,000 | 150 | 200 | 500 | 5,000 | 1.33 |
| 30,000,000 | 150 | 200 | 1000 | 5,000 | 1.17 |
| 40,000,000 | 150 | 200 | 500 | 10,000 | 1.67 |
| 40,000,000 | 150 | 200 | 1000 | 10,000 | 1.33 |
| 40,000,000 | 150 | 300 | 500 | 10,000 | 1.50 |
| 40,000,000 | 150 | 300 | 1000 | 10,000 | 1.00 |

In some cases, the methods and materials provided herein can be used for the early detection of cancer or to stratify tumors on the basis of, for example, genome wide aneuploidy events and, in the target enriched regions: copy number alterations, mutations, and diverse structural variants. In some cases, the methods and materials provided herein can be used to monitor recurrence of cancer following treatment (e.g., surgery) with the enriched (e.g., amplified and/or captured) selected regions being selected based on the SNPs or translocations of the original tumor.

Any appropriate genome can be assessed using the methods and materials provided herein. For example, the genome of a human, horse, bovine species, dog, cat, or monkey can be assessed using the methods and materials provided herein. In addition, any appropriate sample containing genomic nucleic acid can be used as described herein. For example, the methods and materials provided herein can be used to analyze DNA extracted from cells or cell-free DNA extracted from blood, from brushings, or tampons. In some cases, the methods and materials described herein can be used to assess nucleic acid from fresh samples, frozen samples, or formalin-fixed paraffin embedded samples. Any appropriate sample preparation technique can be used to extract DNA from cells or extract cell-free DNA from blood, feces, urine, tampons, or brushing samples. For example, a nucleic acid extraction kits can be used.

Any appropriate genome region can be a selected target region that is amplified or enriched to increase its sequence read coverage during low coverage whole genome sequencing. For example, any one or more of the nucleic acid regions set forth in Table 2 (or portions thereof) can be amplified as described herein to generate amplified selected regions that provide an increased sequence read coverage during low coverage whole genome sequencing. Such nucleic acid regions can be used to detect a genetic defect or element within the amplified regions.

**Table 2. Exemplary selected regions of human genome for amplification or capture enrichment.**

| **Gene** | **Chr** | **Exon** | **Exon Start** | **Exon End** | **Primer Start** | **Primer End** | **Len** | **Fwd Primer** | **SEQ ID NO:** | **Rev Primer** | **SEQ ID NO:** | **ID** |
|---|---|---|---|---|---|---|---|---|---|---|---|---|
| CCND1 | chr11 | 1 | 69455872 | 69456279 | 69455842 | 69456390 | 549 | GGCTTTGATCTTTGCTTAA C | 9 | AAACTTCAAAGTTCTAG CGG | 162 | 1 |
| CCND1 | chr11 | 2 | 69457798 | 69458014 | 69457592 | 69458125 | 534 | GGACTTTCCCTTTCAGTTT C | 10 | AGGAGCAGATATGTCAG AGG | 163 | 2 |
| CCND1 | chr11 | 3 | 69458599 | 69458759 | 69458336 | 69458863 | 528 | GGAGGTCTTTTTGTTTCCA C | 11 | GACATCTTCCCAGACAG CAC | 164 | 3 |
| CCND1 | chr11 | 4 | 69462761 | 69462910 | 69462512 | 69463092 | 581 | TTCCTTGGTTATGTTTGAG TC | 12 | TCTAGGAGCAGTGGAAG AAG | 165 | 4 |
| CCND1 | chr11 | 5 | 69465885 | 69469242 | 69465779 | 69466337 | 559 | TTGCTCTTATAAAGGCTTC C | 13 | TATCATCTGTAGCACAA CCC | 166 | 5 |
| CCND1 | chr11 | 5 | 69465885 | 69469242 | 69466159 | 69466730 | 572 | AAGCTTCATTCTCCTTGTT G | 14 | ACGCTACTGTAACCAAG AGG | 167 | 6 |
| CCND1 | chr11 | 5 | 69465885 | 69469242 | 69466597 | 69467101 | 505 | GCATCTCTGTACTTTGCTT G | 15 | AACAGCGCTATTTCCTA CAC | 168 | 7 |
| CCND1 | chr11 | 5 | 69465885 | 69469242 | 69467056 | 69467580 | 525 | ATTTCCAAGCACTTTCAGT C | 16 | AGAAGGTTTGTGTGTGT GTG | 169 | 8 |
| CCND1 | chr11 | 5 | 69465885 | 69469242 | 69467560 | 69468087 | 528 | ACACACACACACACAAACCT TC | 17 | CAGCAAACAATGTGAAA GAG | 170 | 9 |
| CCND1 | chr11 | 5 | 69465885 | 69469242 | 69468041 | 69468490 | 450 | GGAAATATTCACATCGCT TC | 18 | ACTACTATGATGCTACG CCC | 171 | 10 |
| CCND1 | chr11 | 5 | 69465885 | 69469242 | 69468254 | 69468737 | 484 | TGTTTCACAATACCTCATG C | 19 | GATTTGGAGTCTCTTTA AATTAGC | 172 | 14 |
| CCND1 | chr11 | 5 | 69465885 | 69469242 | 69468591 | 69469036 | 446 | ACCTGTAGGACTCTCATT CG | 20 | TCTCGATACACACAACA TCC | 173 | 13 |
| CCND1 | chr11 | 5 | 69465885 | 69469242 | 69469013 | 69469596 | 584 | TCCTGGATGTTGTGTGTAT C | 21 | AGCCTGCAAATTATTCT CTG | 174 | 12 |
| LMO1 | chr11 | 1 | 8289973 | 8290182 | 8289734 | 8290333 | 600 | GAGACTTCCTAATCCCGC CG | 22 | CTCTGCTGAGGCGAGTA CGG | 175 | 11 |
| LMO1 | chr11 | 2 | 8251837 | 8252051 | 8251723 | 8252126 | 404 | GAGAGGACACACAGGGT ACT | 23 | ATTCTTGGGGGATATTC CTT | 176 | 15 |
| LMO1 | chr11 | 3 | 8248521 | 8248647 | 8248278 | 8248787 | 510 | TATTCACACAGAAATGTG CC | 24 | TCTTATCCTATTGCCTGA GC | 177 | 16 |
| LMO1 | chr11 | 4 | 8245850 | 8246268 | 8245819 | 8246368 | 550 | AGGTCTGTGTCAGTCATG TG | 25 | ACATAGCTCACCTCATA GGC | 178 | 17 |
| MDM2 | chr12 | 1 | 69201951 | 69202271 | 69201702 | 69202276 | 575 | GGCTAAAGGAGTGTCACA GC | 26 | AGTACCTGCTCCTCACC ATC | 179 | 18 |
| MDM2 | chr12 | 2 | 69202987 | 69203072 | 69202745 | 69203311 | 567 | AAGTCCTGACTTGTCTCC AG | 27 | CACGCTTAACAATGTAA TGG | 180 | 19 |
| MDM2 | chr12 | 3 | 69207333 | 69207408 | 69207149 | 69207681 | 533 | TGGATTGGATACTGTCTG TG | 28 | ATTCTGGGAAGGAGTCT ACC | 181 | 20 |
| MDM2 | chr12 | 4 | 69210591 | 69210725 | 69210331 | 69210882 | 552 | TTAGTAGAGATGGGACCA GG | 29 | GGTTCTCAAATAATATG CCG | 182 | 21 |
| MDM2 | chr12 | 5 | 69214104 | 69214154 | 69213983 | 69214509 | 527 | TTTGAATGTGTGCAGTAG TTC | 30 | TCCTTACACATGGTCCT ACC | 183 | 22 |
| MDM2 | chr12 | 6 | 69218142 | 69218210 | 69218039 | 69218363 | 325 | AAATTGCATAAGGGTTTG TG | 31 | TTCTCTTCCTGAAGCTCT TG | 184 | 23 |
| MDM2 | chr12 | 7 | 69218334 | 69218431 | 69218161 | 69218640 | 480 | CATCTGTGAGTGAGAACA GG | 32 | GTAAACTGTGCCTGCTG TAG | 185 | 24 |
| MDM2 | chr12 | 8 | 69222550 | 69222711 | 69222304 | 69222899 | 596 | AGATTGTGCCTCTGTACTC C | 33 | ATTTCTCACAATACCTTG GG | 186 | 25 |
| MDM2 | chr12 | 9 | 69229608 | 69229764 | 69229556 | 69230130 | 575 | ACAGAGGTCAAGAGGTGA TG | 34 | TGGGAAACAGATCTCTA AGG | 187 | 26 |
| MDM2 | chr12 | 10 | 69230451 | 69230529 | 69230398 | 69230878 | 481 | TCTGATTGAAGGAAATAG GG | 35 | GCCTGTAATTCCAGCTA CTC | 188 | 27 |
| MDM2 | chr12 | 11 | 69233053 | 69239324 | 69232933 | 69233478 | 546 | AAACACTGAATATTGAGC CC | 36 | TGACAAATCACACAAGG TTC | 189 | 28 |
| MDM2 | chr12 | 11 | 69233053 | 69239324 | 69233263 | 69233839 | 577 | CAGAGAGTCATGTGTTGA GG | 37 | AGTTGGTGTAAAGGATG AGC | 190 | 29 |
| MDM2 | chr12 | 11 | 69233053 | 69239324 | 69233819 | 69234364 | 546 | AGCTCATCCTTTACACCA AC | 38 | GCTAGATCATGACACTG CAC | 191 | 30 |
| MDM2 | chr12 | 11 | 69233053 | 69239324 | 69234347 | 69234878 | 532 | GCAGTGTCATGATCTAGC AG | 39 | TGAGGTGAGTAGATCAC TTGAG | 192 | 31 |
| MDM2 | chr12 | 11 | 69233053 | 69239324 | 69234715 | 69235284 | 570 | TCTGGGTTCAAGCTATTCT C | 40 | TTTGTCTTACGGGTAAA TGG | 193 | 32 |
| MDM2 | chr12 | 11 | 69233053 | 69239324 | 69235142 | 69235665 | 524 | GCTAAGTAGGATTACAGG CG | 41 | GCTTGAGAGGAAGTCAA GAG | 194 | 33 |
| MDM2 | chr12 | 11 | 69233053 | 69239324 | 69235413 | 69235862 | 450 | TAAAGTACCTTCTTGGCCT G | 42 | ACAGAATGCTTTAGTCC ACC | 195 | 34 |
| MDM2 | chr12 | 11 | 69233053 | 69239324 | 69235711 | 69236286 | 576 | GTGTTAGTTTCTTTGGGAC C | 43 | GTAATCACCTTTCATCG GAG | 196 | 35 |
| MDM2 | chr12 | 11 | 69233053 | 69239324 | 69236212 | 69236802 | 591 | CTCCTTTGGAGACTTAGA ACC | 44 | AGCTTGTTCTACCAGGA ATG | 197 | 36 |
| MDM2 | chr12 | 11 | 69233053 | 69239324 | 69236522 | 69237080 | 559 | AAGGGAGGATATAAGGA ACC | 45 | CTCTCAATAAATGGCCA AAG | 198 | 37 |
| MDM2 | chr12 | 11 | 69233053 | 69239324 | 69237017 | 69237603 | 587 | CCAAATAATGCTTTGAGG AC | 46 | AAAGAGATTCTGCTTGG TTG | 199 | 38 |
| MDM2 | chr12 | 11 | 69233053 | 69239324 | 69237424 | 69237893 | 470 | GGACTGAGGTAATTCTGC AC | 47 | CCCATAAACATGTTGAA TCC | 200 | 39 |
| MDM2 | chr12 | 11 | 69233053 | 69239324 | 69237579 | 69238177 | 599 | AGCTACAACCAAGCAGAA TC | 48 | TGCAACATCATTCTCTC AAG | 201 | 40 |
| MDM2 | chr12 | 11 | 69233053 | 69239324 | 69237775 | 69238260 | 486 | TTCTGAGGAGTATCGGTA GC | 49 | ACCATTCACGATCACTT AGG | 202 | 41 |
| MDM2 | chr12 | 11 | 69233053 | 69239324 | 69238214 | 69238663 | 450 | CTTCTCTTAGGTCACATGG C | 50 | AAGCAGAACCACTTGAA CAC | 203 | 42 |
| MDM2 | chr12 | 11 | 69233053 | 69239324 | 69238402 | 69238927 | 526 | TTGTGAGGCACAAATGTA AG | 51 | TTCACAATGCCATTAAC AAC | 204 | 43 |
| MDM2 | chr12 | 11 | 69233053 | 69239324 | 69238879 | 69239450 | 572 | GGTCTGTAGGCTTATGAT GG | 52 | GAGATGTGGGATTGTAG GAC | 205 | 44 |
| MDM4 | chr1 | 1 | 204485506 | 204485637 | 204485352 | 204485901 | 550 | AAATCTGACGACTTTCAA CC | 53 | ACGTCGACTTTAGGTTT GTC | 206 | 45 |
| MDM4 | chr1 | 2 | 204494611 | 204494724 | 204494451 | 204495019 | 569 | AAGATATGCAGAACCTCA GC | 54 | CATAATTCACTGCAGCT TTG | 207 | 46 |
| MDM4 | chr1 | 3 | 204495487 | 204495562 | 204495232 | 204495823 | 592 | AAATTACCTGGATATGGT GG | 55 | GTCAGGAGACTGAGACC ATC | 208 | 47 |
| MDM4 | chr1 | 4 | 204499811 | 204499945 | 204499574 | 204500079 | 506 | ATCAGTTCATTTCTGTGCT G | 56 | TGCCTCATAGGCTACCT AAC | 209 | 48 |
| MDM4 | chr1 | 5 | 204501318 | 204501374 | 204501252 | 204501832 | 581 | GGCAAACCACTGATATCT TC | 57 | GAGACATATCAACCAAA GGC | 210 | 49 |
| MDM4 | chr1 | 6 | 204506557 | 204506625 | 204506510 | 204506840 | 331 | ATGGTTATTACCAGGGAA GG | 58 | AGAAGTGCTACATCCCA AAG | 211 | 50 |
| MDM4 | chr1 | 7 | 204507336 | 204507436 | 204507222 | 204507638 | 417 | TTCTTGTGTGTAACCCATT G | 59 | ATCCTAGTACTCACGGG TTG | 212 | 51 |
| MDM4 | chr1 | 8 | 204511911 | 204512072 | 204511725 | 204512265 | 541 | TGAAGTCTAAACAAGGGA GG | 60 | AACTGAAGTTGGGCATT TAG | 213 | 52 |
| MDM4 | chr1 | 9 | 204513662 | 204513812 | 204513529 | 204514082 | 554 | GTCCACTGAATAAAGGCA AG | 61 | TACCTTGTTAGCAAAGG GAG | 214 | 53 |
| MDM4 | chr1 | 10 | 204515924 | 204516005 | 204515663 | 204516246 | 584 | TATGGGCATCTTCTCTCTT C | 62 | CAGAGGCATTTATCTCA TCC | 215 | 54 |
| MDM4 | chr1 | 11 | 204518240 | 204527248 | 204518078 | 204518653 | 576 | AAAGACTTTCCTTCATGT GG | 63 | AAGCTACATGGCTTCAA GAG | 216 | 55 |
| MDM4 | chr1 | 11 | 204518240 | 204527248 | 204518561 | 204519094 | 534 | AAGCATGGGAGAACAGTT AG | 64 | AAATGTGCATGGAAGAA ATC | 217 | 56 |
| MDM4 | chr1 | 11 | 204518240 | 204527248 | 204519011 | 204519570 | 560 | TACTTTATGCAGCAGTCA GG | 65 | CTATAATCCCAGCAATT TGG | 218 | 57 |
| MDM4 | chr1 | 11 | 204518240 | 204527248 | 204519551 | 204520101 | 551 | CCAAATTGCTGGGATTAT AG | 66 | AAGACATGTTCTGACGG AAG | 219 | 58 |
| MDM4 | chr1 | 11 | 204518240 | 204527248 | 204519982 | 204520495 | 514 | CCCTGGGACTATAGATTT AGC | 67 | ATGACTCCTAAGACGCA AAG | 220 | 59 |
| MDM4 | chr1 | 11 | 204518240 | 204527248 | 204520474 | 204521069 | 596 | CTCTTTGCGTCTTAGGAGT C | 68 | GTGGTCCAAGACAATTC TTC | 221 | 60 |
| MDM4 | chr1 | 11 | 204518240 | 204527248 | 204520897 | 204521454 | 558 | TGCAGAGACTGATCTTTG AG | 69 | ACCAACAACGACATTAT GAG | 222 | 61 |
| MDM4 | chr1 | 11 | 204518240 | 204527248 | 204521434 | 204521966 | 533 | TCTCATAATGTCGTTGTTG G | 70 | GTAAAGATGAAATTCGG CTC | 223 | 62 |
| MDM4 | chr1 | 11 | 204518240 | 204527248 | 204521808 | 204522394 | 587 | TTGATCCTAAATTTGACA CATC | 71 | GCCTTGCTTTAGTTTAGT GG | 224 | 63 |
| MDM4 | chr1 | 11 | 204518240 | 204527248 | 204522261 | 204522731 | 471 | AAAGTGCTGAGATTACAG GC | 72 | TGGTAATGTGGTGTGAT TTC | 225 | 64 |
| MDM4 | chr1 | 11 | 204518240 | 204527248 | 204522686 | 204523254 | 569 | GCAACGTGCTGTAGACTA TG | 73 | ATTGCATTGAATTGACA CAC | 226 | 65 |
| MDM4 | chr1 | 11 | 204518240 | 204527248 | 204523103 | 204523650 | 548 | CAAGCATTTGAAATATGC AG | 74 | TCACGTTTGGTACATGA GAC | 227 | 66 |
| MDM4 | chr1 | 11 | 204518240 | 204527248 | 204523496 | 204524044 | 549 | TTAGTTCTGATGGTTCTCC C | 75 | TGCTGTATTCACCAATA ACG | 228 | 67 |
| MDM4 | chr1 | 11 | 204518240 | 204527248 | 204523931 | 204524513 | 583 | TATAGGAGCCATTGGATT TC | 76 | GTCAGGAGATCAAGACC ATC | 229 | 68 |
| MDM4 | chr1 | 11 | 204518240 | 204527248 | 204524182 | 204524677 | 496 | ATCTGAAATCCAAGATGC TG | 77 | TACAGCAACTGCTCTGA AAG | 230 | 69 |
| MDM4 | chr1 | 11 | 204518240 | 204527248 | 204524537 | 204525135 | 599 | TCCCAAAGTACTGGGATT AC | 78 | ATTTGCTACTGTTGACA GGG | 231 | 70 |
| MDM4 | chr1 | 11 | 204518240 | 204527248 | 204525034 | 204525491 | 458 | ATTTCTTATCTGAAGGCA CTG | 79 | CATCACACACAGAAAGG AAG | 232 | 71 |
| MDM4 | chr1 | 11 | 204518240 | 204527248 | 204525312 | 204525853 | 542 | TACCAAAGACCCTTATCA GC | 80 | TTCTGTAAGAAGGAAGC CTG | 233 | 72 |
| MDM4 | chr1 | 11 | 204518240 | 204527248 | 204525814 | 204526369 | 556 | TGTCTCAAAGAAATTGAG GTC | 81 | AGTAATCAAACAGGCTC TGC | 234 | 73 |
| MDM4 | chr1 | 11 | 204518240 | 204527248 | 204526066 | 204526663 | 598 | TAAGTGCCTCTTGGGTAG AG | 82 | AGCTACTTGAGAGGTTG AGG | 235 | 74 |
| MDM4 | chr1 | 11 | 204518240 | 204527248 | 204526557 | 204527101 | 545 | GTCTTACTCTGTCACCCAG G | 83 | CTTTCCTCATCTAGTGAG CTG | 236 | 75 |
| MDM4 | chr1 | 11 | 204518240 | 204527248 | 204526920 | 204527482 | 563 | TCAGAGAATCACAAGAGC AG | 84 | GATGGATTTCTTCAGGA TTG | 237 | 76 |
| MYC | chr8 | 1 | 128748314 | 128748869 | 128748285 | 128748719 | 435 | CTTTATAATGCGAGGGTC TG | 85 | TTGTAAGTTCCAGTGCA AAG | 238 | 77 |
| MYC | chr8 | 1 | 128748314 | 128748869 | 128748485 | 128748945 | 461 | GTAGTAATTCCAGCGAGA GG | 86 | ATTTAGGCATTCGACTC ATC | 239 | 78 |
| MYC | chr8 | 2 | 128750493 | 128751265 | 128750452 | 128750908 | 457 | TTTAACTCAAGACTGCCT CC | 87 | TACAGTCCTGGATGATG ATG | 240 | 79 |
| MYC | chr8 | 2 | 128750493 | 128751265 | 128750834 | 128751381 | 548 | ACATGGTGAACCAGAGTT TC | 88 | TCCAGATCTGCTATCTCT CC | 241 | 80 |
| MYC | chr8 | 3 | 128752641 | 128753680 | 128752528 | 128752893 | 366 | GTCCAGAGACCTTTCTAA CG | 89 | TGATCTGTCTCAGGACT CTG | 242 | 88 |
| MYC | chr8 | 3 | 128752641 | 128753680 | 128752715 | 128753285 | 571 | AGAGTCTGGATCACCTTC TG | 90 | TTTGATCATGCATTTGA AAC | 243 | 86 |
| MYC | chr8 | 3 | 128752641 | 128753680 | 128753173 | 128753687 | 515 | AACTTGAACAGCTACGGA AC | 91 | TCACAACTTAAGATTTG GCTC | 244 | 87 |
| MYCL | chr1 | 1 | 40367479 | 40367687 | 40367327 | 40367715 | 389 | AGCGAGTTCAAAGCAAAC TT | 92 | GCGACGAGATATAAGGC AGT | 245 | 81 |
| MYCL | chr1 | 2 | 40366610 | 40367115 | 40366514 | 40367080 | 567 | AGAGCTTGAGAAGAGCCA AT | 93 | TTTCTACGACTATGACT GCG | 246 | 82 |
| MYCL | chr1 | 2 | 40366610 | 40367115 | 40367010 | 40367346 | 337 | ATTTCTTCCAGATGTCCTC G | 94 | AAGTTTGCTTTGAACTC GCT | 247 | 83 |
| MYCL | chr1 | 3 | 40361095 | 40363642 | 40360973 | 40361525 | 553 | GAGTGGAATGACCAGGTT AG | 95 | ATGGTTTCTTTCTGAGGT TG | 248 | 84 |
| MYCL | chr1 | 3 | 40361095 | 40363642 | 40361453 | 40362039 | 587 | AGGGTAGAGAGGCTATTT CC | 96 | TTTGAAGTTCTTCTGGA ACC | 249 | 85 |
| MYCL | chr1 | 3 | 40361095 | 40363642 | 40362026 | 40362521 | 496 | AGAAGAACTTCAAACTTG CC | 97 | CATTGACCATTACCTCA CTG | 250 | 89 |
| MYCL | chr1 | 3 | 40361095 | 40363642 | 40362463 | 40362896 | 434 | TAAAGGTTTCCAACTCCTT G | 98 | AATAAAGGCTTGCATTC TTG | 251 | 90 |
| MYCL | chr1 | 3 | 40361095 | 40363642 | 40363271 | 40363855 | 585 | CCAGGAAGTTGTGATTCT TC | 99 | TTTCCTTCTTGCTAATGT CC | 252 | 91 |
| MYCN | chr2 | 1 | 16080559 | 16081175 | 16080527 | 16081017 | 491 | TTTTTATGGAAATCAGGA GG | 100 | ACCCAGAGATGGTTTTG TTT | 253 | 92 |
| MYCN | chr2 | 1 | 16080559 | 16081175 | 16080642 | 16081165 | 524 | GTTAATAATATCCCCCGA GC | 101 | ACAGCTCAAACACAGAC AGA | 254 | 93 |
| MYCN | chr2 | 1 | 16080559 | 16081175 | 16081147 | 16081538 | 392 | CTGTCTGTGTTTGAGCTGT C | 102 | AACAACAGACACCCATA TCC | 255 | 94 |
| MYCN | chr2 | 2 | 16082069 | 16082976 | 16081882 | 16082346 | 465 | AGCTTGTACACAAAAGGA GG | 103 | CAAACTTCTTCCAGATG TCC | 256 | 95 |
| MYCN | chr2 | 2 | 16082069 | 16082976 | 16082241 | 16082780 | 540 | CTCGAGTTTGACTCGCTA CA | 104 | GTTCACGGGAAAGGGGA AGA | 257 | 96 |
| MYCN | chr2 | 2 | 16082069 | 16082976 | 16082425 | 16082985 | 561 | AGATGCTGCTTGAGAACG AG | 105 | GGTCTTTACCTGAATCG CTC | 258 | 97 |
| MYCN | chr2 | 3 | 16085614 | 16087129 | 16085471 | 16086069 | 599 | ACATCTATGTTGATGGAC CC | 106 | CTCATTCTTTACCAACTC CG | 259 | 98 |
| MYCN | chr2 | 3 | 16085614 | 16087129 | 16086055 | 16086635 | 581 | TTGGTAAAGAATGAGAAG GC | 107 | TGTCAATGGTATTTACA GAA ATG | 260 | 99 |
| MYCN | chr2 | 3 | 16085614 | 16087129 | 16086508 | 16087031 | 524 | GTTCCAAGTTTCCAAACA AC | 108 | AGAACTTTGCATTTACC CAG | 261 | 100 |
| MYCN | chr2 | 3 | 16085614 | 16087129 | 16087008 | 16087449 | 442 | AGAACTGGGTAAATGCAA AG | 109 | TGAGGTCTCAGCTTAAT TCC | 262 | 101 |
| NCOA3 | chr20 | 1 | 46130600 | 46130763 | 46130398 | 46130992 | 595 | AAAAATTAAGGGCAGGGC TA | 110 | AGCTTCGTCTCAGCTCCT AC | 263 | 102 |
| NCOA3 | chr20 | 2 | 46211926 | 46212005 | 46211894 | 46212483 | 590 | AAATTCAATCCCTCCTCTT C | 111 | AGGTGATCTAACCACCT CAG | 264 | 103 |
| NCOA3 | chr20 | 3 | 46250972 | 46251074 | 46250747 | 46251198 | 452 | GGAACATTTCTGTCTTGG AG | 112 | ACTTACCACGAAGTGAA ACC | 265 | 104 |
| NCOA3 | chr20 | 4 | 46252654 | 46252827 | 46252552 | 46253120 | 569 | GTAATCATGTAATAGTGT TGTATAGGG | 113 | GATCTGTCACAGTTTCTC CC | 266 | 105 |
| NCOA3 | chr20 | 5 | 46254124 | 46254225 | 46253918 | 46254512 | 595 | TTAGGTATCTTCTGGCTTC C | 114 | TACAGGCTACCTTTCCTT TC | 267 | 106 |
| NCOA3 | chr20 | 6 | 46255745 | 46255920 | 46255621 | 46256183 | 563 | TTACCTCCTTGAAGGTCTT G | 115 | ATTTCAGGCTGGCAATA TAC | 268 | 107 |
| NCOA3 | chr20 | 7 | 46256304 | 46256493 | 46256232 | 46256752 | 521 | CTTGAATTCTTGATGATG GTC | 116 | TGGTAATAAAGCTCTCA GGG | 269 | 108 |
| NCOA3 | chr20 | 8 | 46256665 | 46256767 | 46256391 | 46256976 | 586 | ATTCTGGAAGACATAAAC GC | 117 | AACATACCCAATTCAAA TGC | 270 | 109 |
| NCOA3 | chr20 | 9 | 46262239 | 46262380 | 46262160 | 46262475 | 316 | CAGTGCTAAGCCATGTGT AG | 118 | TAAATCCAGGAGTTCGA GTC | 271 | 110 |
| NCOA3 | chr20 | 10 | 46262791 | 46262939 | 46262711 | 46263063 | 353 | GTATATTTCCTCCCTGTCC C | 119 | CATCAAACCCAATAACC TTC | 272 | 111 |
| NCOA3 | chr20 | 11 | 46264065 | 46264457 | 46263932 | 46264470 | 539 | CAAAGTGCTGGGAATATA GG | 120 | TCAACACAAATACCTGC AAC | 273 | 112 |
| NCOA3 | chr20 | 12 | 46264634 | 46265506 | 46264235 | 46264834 | 600 | ATGAGTGGAGCTAGGTAT GG | 121 | CTTGGAATCCTGATTGC TTA | 274 | 113 |
| NCOA3 | chr20 | 12 | 46264634 | 46265506 | 46264800 | 46265238 | 439 | CCCAACCAAGTAAAGTAA GC | 122 | GCAGTAATCTTGGCTAC CTC | 275 | 114 |
| NCOA3 | chr20 | 12 | 46264634 | 46265506 | 46265206 | 46265783 | 578 | GAATTCACCAGCTGAGGT AG | 123 | CTCTTAATGACCCAATC TGC | 276 | 115 |
| NCOA3 | chr20 | 13 | 46266391 | 46266527 | 46266333 | 46266855 | 523 | TGTTTATACCTGTGTGTCT GG | 124 | TTAATCCAGTTCTCTGTG GC | 277 | 116 |
| NCOA3 | chr20 | 14 | 46267751 | 46267946 | 46267493 | 46268028 | 536 | AGTTCTCAGTACTTCAGC CG | 125 | CTCCCAATTATTTAGAT GGC | 278 | 117 |
| NCOA3 | chr20 | 15 | 46268320 | 46268566 | 46268163 | 46268576 | 414 | ATAGTGGCCTATGTCTCC AC | 126 | GGACACTTACTCATTTG AAGC | 279 | 118 |
| NCOA3 | chr20 | 16 | 46268668 | 46268795 | 46268503 | 46268943 | 441 | CGGTCTAATAGCATACCA GG | 127 | AGAGTTACACGAGAAAT GCC | 280 | 119 |
| NCOA3 | chr20 | 17 | 46270956 | 46271128 | 46270628 | 46271179 | 552 | AGGAGTATCTTCTCCCAT CC | 128 | GCGCACACACACAAATA TAC | 281 | 120 |
| NCOA3 | chr20 | 18 | 46275816 | 46276110 | 46275557 | 46276087 | 531 | CACAGTACACCTGGTTCT TG | 129 | GAAGCTGCATTCTAAGT TGC | 282 | 121 |
| NCOA3 | chr20 | 18 | 46275816 | 46276110 | 46275868 | 46276458 | 591 | GTAATGATGGATCAGAAG GC | 130 | AAATGCTGAAATCAAGA AGG | 283 | 122 |
| NCOA3 | chr20 | 19 | 46277748 | 46277853 | 46277654 | 46278204 | 551 | GATATTACCTCATTGGCT GG | 131 | TGCATGTTGTTTCATAAT CC | 284 | 123 |
| NCOA3 | chr20 | 20 | 46279728 | 46280020 | 46279700 | 46280285 | 586 | TAATTGCACTCTTTCTTGG G | 132 | AACTTTGCAGTGTTTCTT CC | 285 | 124 |
| NCOA3 | chr20 | 21 | 46281149 | 46281324 | 46281096 | 46281383 | 288 | TTCTAAGGAGAAGGCATT TG | 133 | TAAGTTCTTGGACTTCTG GG | 286 | 125 |
| NCOA3 | chr20 | 22 | 46281674 | 46281816 | 46281629 | 46282021 | 393 | GCTAAAGTGACTTCCAGA GG | 134 | GAGATCCCATCTTACAA TGC | 287 | 126 |
| NCOA3 | chr20 | 23 | 46282149 | 46285621 | 46282008 | 46282592 | 585 | TAAGATGGGATCTCAGGA AC | 135 | TCTTTGTCCAATACTGCA AC | 288 | 127 |
| NCOA3 | chr20 | 23 | 46282149 | 46285621 | 46282430 | 46282949 | 520 | ATTCTGGAGACATGGAGT GT | 136 | AACCAGGAATGTGTTTC ACT | 289 | 128 |
| NCOA3 | chr20 | 23 | 46282149 | 46285621 | 46282912 | 46283260 | 349 | TTGAGGTCTTGAGGGAAT AG | 137 | ACCACACAGCTTACTGA AATC | 290 | 129 |
| NCOA3 | chr20 | 23 | 46282149 | 46285621 | 46283242 | 46283793 | 552 | TTTCAGTAAGCTGTGTGG TG | 138 | AGGGACATAATGAAAGC ATC | 291 | 130 |
| NCOA3 | chr20 | 23 | 46282149 | 46285621 | 46283688 | 46284229 | 542 | GACCTGAATCCCATATTG AG | 139 | GTGGGTCTGGAAATAAT CAG | 292 | 131 |
| NCOA3 | chr20 | 23 | 46282149 | 46285621 | 46284210 | 46284671 | 462 | CTGATTATTTCCAGACCC AC | 140 | AGAAATCTTGAGTTTGC ACC | 293 | 139 |
| NCOA3 | chr20 | 23 | 46282149 | 46285621 | 46284324 | 46284768 | 445 | AAATCCGAAAACTTCCAT TG | 141 | GAGGAGAGGTAGACAG CAGG | 294 | 137 |
| NCOA3 | chr20 | 23 | 46282149 | 46285621 | 46284746 | 46285291 | 546 | ACTCCTGCTGTCTACCTCT C | 142 | TGCTCCTAGGAACCTAA TTG | 295 | 138 |
| NCOA3 | chr20 | 23 | 46282149 | 46285621 | 46285161 | 46285693 | 533 | AGTTCTTTGATCCAGAGG TG | 143 | TTCCTTAACCTCCTTTAC CC | 296 | 132 |
| NKX2-1 | chr14 | 1 | 36989257 | 36989430 | 36989105 | 36989609 | 505 | AGGAGAGATGGTTGAGAG GA | 144 | ACTGAAAAACCCCTGAG CTG | 297 | 133 |
| NKX2-1 | chr14 | 2 | 36988189 | 36988575 | 36987990 | 36988496 | 507 | GCTACCAAGTGCCTGTTC TT | 145 | AGCTACAAGAAAGTGGG CAT | 298 | 134 |
| NKX2-1 | chr14 | 2 | 36988189 | 36988575 | 36988246 | 36988667 | 422 | TTCCTCATGGTGTCCTGGT A | 146 | ACCAGAATATTTGGCAA AGG | 299 | 135 |
| NKX2-1 | chr14 | 3 | 36985603 | 36987225 | 36985377 | 36985969 | 593 | ACTGCTCAAGATTTGTTTC C | 147 | TCACTGACACAAAGGAA GTG | 300 | 136 |
| NKX2-1 | chr14 | 3 | 36985603 | 36987225 | 36985737 | 36986227 | 491 | TACACAGATTTGTCAATG CC | 148 | ATCTTTAAGCAGAGAAG GGC | 301 | 140 |
| NKX2-1 | chr14 | 3 | 36985603 | 36987225 | 36986160 | 36986513 | 354 | GAAAACCCATTTGAATCA CC | 149 | CTCCACCTTGCTATACG GTC | 302 | 141 |
| NKX2-1 | chr14 | 3 | 36985603 | 36987225 | 36986374 | 36986970 | 597 | TGTTAAGAAAAGTCGAAG CG | 150 | AGAACCACCGCTACAAA ATG | 303 | 142 |
| NKX2-1 | chr14 | 3 | 36985603 | 36987225 | 36986967 | 36987556 | 590 | TTCTGGAACCAGATCTTG AC | 151 | TAATCCTAATGCTCTGA CCC | 304 | 143 |
| SKP2 | chr5 | 1 | 36152144 | 36152372 | 36152137 | 36152620 | 484 | GAAACTACAATTCCCAGC AG | 152 | GAGAGACAGGGCAATC ATAC | 305 | 144 |
| SKP2 | chr5 | 2 | 36152872 | 36153144 | 36152615 | 36153148 | 534 | TCTCTCTCCTTGTCTGTTC C | 153 | TTACCTGGAAAGTTCTC TCG | 306 | 145 |
| SKP2 | chr5 | 3 | 36163746 | 36163858 | 36163699 | 36164087 | 389 | GATAGGGTGAAAGAATGG TG | 154 | ACTGAATACAGGGCAAA GAG | 307 | 146 |
| SKP2 | chr5 | 4 | 36166620 | 36166764 | 36166514 | 36167017 | 504 | GCTTCAAGGAGATTTAGC AG | 155 | AAGACAAATGTGCCTCT TTC | 308 | 147 |
| SKP2 | chr5 | 5 | 36168414 | 36168549 | 36168352 | 36168852 | 501 | GTTTGAAATTGGATGTAC CC | 156 | CAGCATTCACTAACAAG GTG | 309 | 148 |
| SKP2 | chr5 | 6 | 36170445 | 36170544 | 36170281 | 36170703 | 423 | GAGGCAAATTATCCTGTT TG | 157 | TTGGACAGAAAGTTAGG AGG | 310 | 149 |
| SKP2 | chr5 | 7 | 36171704 | 36171835 | 36171414 | 36171948 | 535 | AAGACTGGCATTTCTACC TG | 158 | CATGCACTGGATTAAAT GAG | 311 | 150 |
| SKP2 | chr5 | 8 | 36177066 | 36177118 | 36176945 | 36177324 | 380 | GTGTGGTTCTAATTGCATT G | 159 | ATTCCTGAAAGCAGTCA TTC | 312 | 151 |
| SKP2 | chr5 | 9 | 36177286 | 36177394 | 36177180 | 36177543 | 364 | GGGAAAGGATCATAATGT TG | 160 | CTCTGCTGGTCTTTCATA GC | 313 | 152 |
| SKP2 | chr5 | 10 | 36183941 | 36184142 | 36183823 | 36184304 | 482 | TGCCTTTATCTGCTTAGAC C | 161 | CAAGCATATGAAGTAGA TGGG | 314 | 153 |

In some cases, amplification primers designed to amplify a portion of a human genome targeted by one or more of the FISH probes (e.g., a FISH probe set forth in Table 3) can be used in a single assay as described herein. For example, amplification primers designed to amplify a portion of a human genome targeted by 5, 10, 20, or more FISH probes can be used in a single assay as described herein. In some cases, two or more different amplification primer pairs can be designed to amplify different portions of the same region of a human genome targeted by one of a FISH probe. For example, three primer pairs can be designed to amplify three different regions of the first FISH probe listed in Table 3. In some cases, as described herein, nucleic acid capture techniques can be used in addition to or in place of amplification techniques to increase sequence read coverage.

The invention will be further described in the following examples, which do not limit the scope of the invention described in the claims.

### EXAMPLES

### Example 1 - Combining LC-WGS and targeted nucleic acid amplification to improve the interpretation of Cancer Panel Tests

The combination of LC-WGS and targeted nucleic acid amplification is used to improve the clinical interpretation of Cancer Panel Tests that focus primarily on identifying mutations driving tumorgenesis in targeted regions of the genome. LC-WGS provides information in the genome wide nature and location of amplifications and deletions. This information is used to assess the aggressiveness of the tumor and/or provide additional support to the mutations reported in the targeted regions.

The values of combining LC-WGS and targeted nucleic acid amplification was highlighted by performing the following. Whole DNA of biospecimens was extracted. Targeted regions amplification was performed using an amplicon-based protocol to allow variant calling. The targeted amplification was performed using a number of cycles that was protocol specific, but might vary from protocol to protocol (15 to 20 cycles). The amplification was done below saturation level, leaving in solution about 25% of reads that do not map to the target regions but map the remaining areas of the genome. Upon sequencing, 3.5M reads in total were obtained. The 2.6M reads mapping the target regions were extracted and processed for variant calling using a DNA processing workflow. The high coverage of these regions (at about 1000x average coverage) allowed for clinical grade variants calling. For the two ovarian samples displayed on Figures 4A and 4B, mutations in the DNA repair and signal transduction genes were reported in the clinical report.

The 0.9M remaining reads (not mapped to these targeted regions) were processed. The resulting aligned reads were clustered in 10kb bins. The count of the reads in each bin was displayed on Figures 4A and 4B for two ovarian tumor samples. The plots clearly highlighted chromosome level amplifications and target local amplifications that can be used to further refine the interpretation of the mutations. For example, the balance of chromosome or chromosome arm amplification and local amplification can be informative of the aggressiveness of the tumor.

### Example 2 - Combining LC-WGS and targeted nucleic acid amplification to replace a FISH assay

FISH assays are commonly used by clinical laboratories to report the presence of cancer cells in cytology specimens. For example, the UroVysion FISH assay (Abbott Molecular Inc.) is used to identify cancer cells in urine and biliary samples. This FISH assay includes a set of four fluorescent probes that target the chromosomal location 9p21 and the centromeres of chromosomes 3, 7, and 17. Probes targeting chromosomal locations are used to report amplifications and deletions in these regions. The ones targeting centromeres identify the loss or the presence of additional copies of chromosomes.

For lung and pleural samples, the LaVysion FISH assay (Abbott Molecular Inc.) is used. The four fluorescent probes of this assay target chromosomal locations 7p12, 5p12, 8q24, and the centromere of chromosome 6. Each of these FISH probes is greater than 150,000 bases. The probes are of large size to ensure that their luminescence is high enough to be observed under a microscope.

An assay is designed as described herein to identify deletions and/or amplifications in the genomic regions targeted by the FISH probes, while also having the ability to provide a low resolution global view of alterations across the genome. The regions amplified by these primers overlap with the ones targeted by the FISH probes. The amplified regions are not the same size as the FISH probes since the FISH probes are often greater than 150,000 bases long for technical reasons that are specific to the FISH assay. The FISH probes that target centromeres identify whole chromosome amplifications and/or deletions, which will be identified by the LC-WGS of the designed assay.

In particular, the designed assay combines both the UroVysion and LaVysion in a single assay as set forth in Table 3. Table 3 provides a list of primers that are used to amplify genomic regions 9p21, 7p12, 5p12, and 8q24. The design of these primers was optimized for a melting temperature of about 60 degrees. Primers for the FISH probes targeting centromere regions were not included since the LC-WGS component of the designed assay can identify genomic amplifications and/or deletions of whole chromosomes. Table 3 provides in the 1st column the cytoband location of the regions amplified by the primers followed by the genomic start and end coordinates of the region amplified by the primers, the length of the amplified genomic region, and the sequence of the forward and reverse primers.

**Table 3. Example of the design of an assay that replaces two FISH assays.**

| Cytoband | start | end | length | forward | reverse |
|---|---|---|---|---|---|
| 9p21 | 26549942 | 26550536 | 595 | GTCTGGTTCTGG CTCTGTGC (SEQ ID NO:1) | GCCACCTCCTCTTT GTCAGC (SEQ ID NO:2) |
| 7p12 | 51867623 | 51868189 | 567 | AAGAGTTGCCA AGGCACGAC (SEQ ID NO:3) | TGACAGGCTTGAA TGCACCC (SEQ ID NO:4) |
| 5p12 | 43864904 | 43865490 | 587 | AGACTTCACCTT TGGTGCCC (SEQ ID NO:5) | CCTGGAGAACAGG ATGCGAC (SEQ ID NO:6) |
| 8q24 | 130915820 | 130916382 | 563 | TTCAACCAACCC ATCAGCGG (SEQ ID NO:7) | TTCATGGCCACCA CAATGGC (SEQ ID NO:8) |

### Example 3 - Single assay for the combined reporting for fetal fraction estimation and the presence to fetal trisomy from the blood of the mother

LC-WGS sequencing has been successfully applied to the detection fetal trisomy from the blood of pregnant women. However, to optimize the selectivity and sensitivity of LC-WGS, an additional test is needed to measure the fetal fraction. This additional test can be implemented using SNP microarrays to measure the allelic imbalance. Some *in silico* approaches (e.g., bioinformatics) also have been used for the same purpose.

An assay is designed as described herein to identify in a single assay both the fetal fraction in the blood of the mother and the presence of fetal trisomy. For this assay, the amplified regions are designed to target SNPs empirically selected to maximize the likelihood to be heterogeneous in the fetus and homozygous in the mother. The ratio of the reads mapped to the major and minor allele is informative of the fraction of the DNA from the fetus present in the blood of the mother. Calling the genotypes of SNPs is not possible from LC-WGS alone since this technique does not have enough reads available to call genotypes.

### Example 4 - Combining LC-WGS and targeted nucleic acid amplification for the early detection of cancer

The methods and materials provided herein are used for the early detection of cancer in cell free DNA. As tumors develop, a significant percentage of tumor cells die, shedding their abnormal DNA in the blood stream. The methods and materials provided herein are used to detect genomic amplification and/or deletion events in cell free DNA, thereby detecting the presence of a tumor. The low coverage whole genome sequencing of the assay provides a low resolution whole genome view of amplifications and/or deletions, while oncogenes frequently observed as being amplified and/or deleted across cancers are assessed at a higher sensitivity level using PCR amplification targeted regions. The following genes, which are frequently amplified across tumor types, are enriched as described herein: CCND1, LMO1, MDM2, MDM4, MYC, MYCL1, MYCN, NCOA3, NKX2-1, and SKP2. With the designed assay, multiple amplicons (e.g., about 5) of about 150 bp in length are assessed for each gene (for a total of about 50 amplicons per assay). Assuming that 400,000 reads of 150 bp is sequenced per sample, if 50 amplicons of 150 bp are used to amplify 50 regions of the genome, then each region exhibits a coverage of about 600x while the LC-WGS maintains an average coverage of about 1x for the DNA not enriched (Table 4).

**Table 4.**

| reads per sample | read length (bp) | number of amplified regions | length of amplified regions | coverage of the amplified regions | LC-WGS coverage |
|---|---|---|---|---|---|
| 30,000,000 | 150 | 50 | 150 | 600x | 1.0 |

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

### EMBODIMENTS

Although the present invention is defined in the claims, it should be understood that the present invention can also (alternatively) be defined in accordance with the following embodiments:
1. A method for increasing the number of sequencing reads of one or more pre-selected genomic regions using low coverage whole genome sequencing, wherein said method comprises performing an amplification reaction using a genomic nucleic acid sample to amplify one or more pre-selected genomic regions, thereby forming an amplified sample, and performing low coverage whole genome sequencing using said amplified sample, wherein the coverage of said pre-selected genomic regions using said low coverage whole genome sequencing is greater than 250x, and wherein the coverage of regions outside said pre-selected genomic regions using said low coverage whole genome sequencing is less than 3x.
2. The method of embodiment 1, wherein said one or more pre-selected genomic regions is from one pre-selected genomic region to 2500 pre-selected genomic regions.
3. The method of embodiment 1, wherein said one or more pre-selected genomic regions is from one pre-selected genomic region to 2000 pre-selected genomic regions.
4. The method of embodiment 1, wherein said one or more pre-selected genomic regions is from one pre-selected genomic region to 1500 pre-selected genomic regions.
5. The method of embodiment 1, wherein said low coverage whole genome sequencing is whole genome sequencing with less than 2x genome wide coverage.
6. The method of embodiment 1, wherein said low coverage whole genome sequencing is whole genome sequencing with less than 1x genome wide coverage.
7. The method of embodiment 1, wherein said genomic nucleic acid sample is a human genomic nucleic acid sample.
8. The method of embodiment 1, wherein the coverage of said pre-selected genomic regions using said low coverage whole genome sequencing is greater than 500x.
9. The method of embodiment 1, wherein the coverage of said pre-selected genomic regions using said low coverage whole genome sequencing is greater than 1000x.
10. The method of embodiment 1, wherein said method comprises performing said amplification reaction using said genomic nucleic acid sample to amplify one or more pre-selected genomic regions having a length from about 150 bp to about 750 bp.
11. A method for increasing the number of sequencing reads of one or more pre-selected genomic regions using low coverage whole genome sequencing, wherein said method comprises performing a nucleic acid capture reaction using a genomic nucleic acid sample to enrich one or more pre-selected genomic regions, thereby forming an enriched sample, and performing low coverage whole genome sequencing using said enriched sample, wherein the coverage of said pre-selected genomic regions using said low coverage whole genome sequencing is greater than 250x, and wherein the coverage of regions outside said pre-selected genomic regions using said low coverage whole genome sequencing is less than 3x.
12. The method of embodiment 11, wherein said one or more pre-selected genomic regions is from one pre-selected genomic region to 2500 pre-selected genomic regions.
13. The method of embodiment 11, wherein said one or more pre-selected genomic regions is from one pre-selected genomic region to 2000 pre-selected genomic regions.
14. The method of embodiment 11, wherein said one or more pre-selected genomic regions is from one pre-selected genomic region to 1500 pre-selected genomic regions.
15. The method of embodiment 11, wherein said low coverage whole genome sequencing is whole genome sequencing with less than 2x genome wide coverage.
16. The method of embodiment 11, wherein said low coverage whole genome sequencing is whole genome sequencing with less than 1x genome wide coverage.
17. The method of embodiment 11, wherein said genomic nucleic acid sample is a human genomic nucleic acid sample.
18. The method of embodiment 11, wherein the coverage of said pre-selected genomic regions using said low coverage whole genome sequencing is greater than 500x.
19. The method of embodiment 11, wherein the coverage of said pre-selected genomic regions using said low coverage whole genome sequencing is greater than 1000x.
20. The method of embodiment 11, wherein said method comprises performing said nucleic acid capture reaction using said genomic nucleic acid sample to capture one or more pre-selected genomic regions having a length from about 150 bp to about 750 bp.

## Claims

1. A method for increasing the number of sequencing reads of one or more pre-selected genomic regions using low coverage whole genome sequencing, wherein said method comprises:
performing a nucleic acid capture reaction using a genomic nucleic acid sample to enrich one or more pre-selected genomic regions, thereby forming an enriched sample, and
performing low coverage whole genome sequencing using said enriched sample,
wherein the coverage of said pre-selected genomic regions using said low coverage whole genome sequencing is greater than 250x,
and wherein the coverage of regions outside said pre-selected genomic regions using said low coverage whole genome sequencing is less than 3x.

2. The method of claim 1, wherein said one or more pre-selected genomic regions is from one pre-selected genomic region to 2500 pre-selected genomic regions.

3. The method of claim 1, wherein said one or more pre-selected genomic regions is from one pre-selected genomic region to 2000 pre-selected genomic regions.

4. The method of claim 1, wherein said one or more pre-selected genomic regions is from one pre-selected genomic region to 1500 pre-selected genomic regions.

5. The method of claim 1, wherein said low coverage whole genome sequencing is whole genome sequencing with less than 2x genome wide coverage.

6. The method of claim 1, wherein said low coverage whole genome sequencing is whole genome sequencing with less than 1x genome wide coverage.

7. The method of claim 1, wherein said genomic nucleic acid sample is a human genomic nucleic acid sample.

8. The method of claim 1, wherein the coverage of said pre-selected genomic regions using said low coverage whole genome sequencing is greater than 500x.

9. The method of claim 1, wherein the coverage of said pre-selected genomic regions using said low coverage whole genome sequencing is greater than 1000x.

10. The method of claim 1, wherein said method comprises performing said nucleic acid capture reaction using said genomic nucleic acid sample to capture one or more pre-selected genomic regions having a length from about 150 bp to about 750 bp.
